# EUROPEAN PATENT APPLICATION

(11) **EP 3 834 627 A1**
(43) Date of publication of application: **16.06.2021**
(21) Application number: 19846844.9
(22) Date of filing: 05.08.2019
(51) Int. Cl.: A23L 33/10, A61K 31/121, A61K 31/353, A61K 31/7048, A61P 7/00, A61P 19/06, A61P 43/00

(54) **COMPOSITION FOR PROMOTING URIC ACID EXCRETION, COMPOSITION FOR INHIBITING URAT1 AND COMPOSITION FOR LOWERING BLOOD URIC ACID LEVEL**

(30) Priority: 10.08.2018 JP 2018151541
(71) Applicant: Suntory Holdings Limited, Osaka 530-8203 (JP)
(72) Inventor: YAMAMURA, Junki, Soraku-gun, Kyoto 619-0284 (JP); TATSU, Sotaro, Soraku-gun, Kyoto 619-0284 (JP)
(74) Representative: Vossius & Partner Patentanwälte Rechtsanwälte mbB
(86) International application number: PCT/JP2019/030731
(87) International publication number: WO 2020/031961

(57) **Abstract**

The present invention aims to provide a composition for promoting uric acid excretion. Specifically, the present invention aims to provide a composition for promoting uric acid excretion, wherein the composition promotes uric acid excretion by URAT1 inhibition, a composition for inhibiting URAT1, a composition for lowering blood uric acid level, a method of promoting uric acid excretion, a method of inhibiting URAT1, and a method of lowering blood uric acid level. The present invention relates to a composition for promoting uric acid excretion and the like, containing at least one flavanone compound selected from the group consisting of a flavanone free of methoxy groups or having one methoxy group and a flavanone glycoside free of methoxy groups or having one methoxy group as an active ingredient.

## Description

### TECHNICAL FIELD

The present invention relates to a composition for promoting uric acid excretion, a composition for inhibiting URAT1, and a composition for lowering blood uric acid level. The present invention also relates to a method of promoting uric acid excretion, a method of inhibiting URAT1, and a method of lowering blood uric acid level. The present invention also relates to use of a flavanone compound for promoting uric acid excretion or for inhibiting URAT1, and use of isoxanthohumol for lowering blood uric acid level.

### BACKGROUND ART

In the human body, for example, uric acid is produced by the breakdown of purines obtained from food. Uric acid is dissolved in body fluids such as blood, circulates in the body, and is excreted into the urine. The amount of uric acid in the body increases in response to a metabolic disorder such as increased production of uric acid, decreased uric acid excretion ability, or the like caused by some reason, resulting in hyperuricemia in some cases. Hyperuricemia is a condition in which the blood uric acid level (serum urate level) is high, and is known to cause gout, urinary stone, and/or kidney disorders, for example.

Therapeutic drugs for hyperuricemia include drugs such as benzbromarone which inhibit the activity of URAT1 (renal urate transporter) so as to promote uric acid excretion, and drugs such as allopurinol which inhibit xanthine oxidase (uric acid synthase) so as to inhibit urate synthesis. These drugs are prescribed according to patients' types of hyperuricemia. While being highly effective, these drugs are often associated with side effects such as liver disorders. In addition, since hyperuricemia is persistent, stopping taking the drug causes recurrence of hyperuricemia. Thus, definitive treatment of hyperuricemia requires long-term intake of the drug.

Therefore, there has been a desire for a component effective in ameliorating hyperuricemia and safe for long-term intake, such as one derived from a natural product which has been allowed for daily intake.

As a means to ameliorate hyperuricemia with a component derived from a natural product, Patent Literature 1 describes a blood uric acid level lowering agent containing xanthohumol as an active ingredient. Patent Literature 2 suggests a xanthine oxidase inhibitor containing a polyphenol fraction derived from apples and/or hops. Patent Literature 3 describes a uric acid excretion promoter containing ellagic acid as an active ingredient. Patent Literature 4 suggests a composition for promoting uric acid excretion which contains, as an active ingredient, a mixture of a liquid obtained by squeezing or crushing *Citrus depressa* peel, a lactic acid fermented product of *Citrus depressa* peel, and a lactic acid fermented product of papaya fruits.

### CITATION LIST

### - Patent Literature

Patent Literature 1: JP 2017-61436 A
Patent Literature 2: JP 2006-169227 A
Patent Literature 3: JP 2006-273762 A
Patent Literature 4: JP 2016-155825 A

### SUMMARY OF INVENTION

### - Technical Problem

Urate transporter 1 (URAT1) is expressed in the proximal convoluted tubule of the kidney. It is a transporter that reabsorbs uric acid. After almost 100% of uric acid in the blood is filtered in glomerulus, a majority is reabsorbed by URAT1 in the tubule, and the rest is excreted into the urine. Thus, inhibiting the activity of URAT1 enhances uric acid excretion during urination. Patent Literatures 1 to 4 nowhere state that a flavanone and a flavanone glycoside which are free of methoxy groups or which have one methoxy group have an action to inhibit URAT1 activity or an action to promote uric acid excretion.

The present invention aims to provide a composition for promoting uric acid excretion, specifically, a composition for promoting uric acid excretion, wherein the composition promotes uric acid excretion by URAT1 inhibition. The present invention also aims to provide a composition for inhibiting URAT1. The present invention also aims to provide a composition for lowering blood uric acid level. The present invention still also aims to provide a method of promoting uric acid excretion, a method of inhibiting URAT1, and a method of lowering blood uric acid level.

### - Solution to Problem

As a result of extensive studies to solve the above problem, the present inventors found that a specific flavanone compound contained in plants such as hops that have been widely used in foods and beverages has URAT1 inhibitory action, and completed the present invention based on the finding.

Specifically, although not limited thereto, the present invention relates to, for example, a composition for promoting uric acid excretion, a composition for inhibiting URAT1, a composition for lowering blood uric acid level, and a method of promoting uric acid excretion, which are described below.
(1) A composition for promoting uric acid excretion, containing: at least one flavanone compound selected from the group consisting of a flavanone free of methoxy groups or having one methoxy group and a flavanone glycoside free of methoxy groups or having one methoxy group, as an effective ingredient.
(2) The composition for promoting uric acid excretion according to (1) above, wherein the flavanone compound is at least one compound selected from the group consisting of isoxanthohumol, naringenin, hesperidin, and eriodictyol.
(3) The composition for promoting uric acid excretion according to (1) or (2) above, wherein the flavanone compound is isoxanthohumol.
(4) The composition for promoting uric acid excretion according to any one of (1) to (3) above, wherein the composition promotes uric acid excretion by URAT1 inhibition.
(5) A composition for inhibiting URAT1, containing:
   at least one flavanone compound selected from the group consisting of a flavanone free of methoxy groups or having one methoxy group and a flavanone glycoside free of methoxy groups or having one methoxy group.
(6) The composition for inhibiting URAT1 according to (5) above, wherein the flavanone compound is at least one compound selected from the group consisting of isoxanthohumol, naringenin, hesperidin, and eriodictyol.
(7) A composition for lowering blood uric acid level, containing: isoxanthohumol as an active ingredient.
(8) The composition for lowering blood uric acid level according to (7) above, wherein the composition is used for preventing or ameliorating hyperuricemia.
(9) The composition for lowering blood uric acid level according to (7) above, wherein the composition is used for preventing or ameliorating gout.
(10) The composition according to any one of (1) to (9) above, wherein the composition is a food or beverage or a pharmaceutical product.
(11) A method of promoting uric acid excretion, including: administering or feeding at least one flavanone compound selected from the group consisting of a flavanone free of methoxy groups or having one methoxy group and a flavanone glycoside free of methoxy groups or having one methoxy group to a subject.
(12) A method of inhibiting URAT1, including: administering or feeding at least one flavanone compound selected from the group consisting of a flavanone free of methoxy groups or having one methoxy group and a flavanone glycoside free of methoxy groups or having one methoxy group to a subject.
(13) A method of lowering blood uric acid level, including: administering or feeding isoxanthohumol to a subject.
(14) Use of at least one flavanone compound for promoting uric acid excretion, the at least one flavanone compound being selected from the group consisting of a flavanone free of methoxy groups or having one methoxy group and a flavanone glycoside free of methoxy groups or having one methoxy group.
(15) Use of at least one flavanone compound for inhibiting URAT1, the at least one flavanone compound being selected from the group consisting of a flavanone free of methoxy groups or having one methoxy group and a flavanone glycoside free of methoxy groups or having one methoxy group.
(16) Use of isoxanthohumol for lowering blood uric acid level.

### - Advantageous Effects of Invention

The present invention provides a composition for promoting uric acid excretion, wherein the composition promotes uric acid excretion by URAT1 inhibition. The present invention also provides a composition for inhibiting URAT1 and a composition for lowering blood uric acid level. The present invention provides a method of promoting uric acid excretion, a method of inhibiting URAT1, and a method of lowering blood uric acid level.

### BRIEF DESCRIPTION OF DRAWINGS

Fig. 1 is a graph showing the URAT1 inhibition rates of isoxanthohumol and xanthohumol.
Fig. 2 shows the URAT1 inhibition rate of naringenin.
Fig. 3 shows the URAT1 inhibition rate of hesperidin.
Fig. 4 shows the URAT1 inhibition rate of eriodictyol.

### DESCRIPTION OF EMBODIMENTS

The composition for promoting uric acid excretion of the present invention contains, as an active ingredient, at least one flavanone compound selected from the group consisting of a flavanone free of methoxy groups or having one methoxy group and a flavanone glycoside free of methoxy groups or having one methoxy group.

The composition for inhibiting URAT1 of the present invention contains, as an active ingredient, at least one flavanone compound selected from the group consisting of a flavanone free of methoxy groups or having one methoxy group and a flavanone glycoside free of methoxy groups or having one methoxy group.

Hereinafter, the flavanone compound used in the present invention is simply referred to as "the flavanone compound". The composition for promoting uric acid excretion and the composition for inhibiting URAT1 of the present invention each may contain one or more flavanone compounds as an active ingredient(s).

The flavanone compound used in the present invention is a flavanone and/or a flavanone glycoside free of methoxy groups or having one methoxy group (having zero or one methoxy group). Such a flavanone and a flavanone glycoside have URAT1 inhibitory action.

Examples of the flavanone free of methoxy groups or having one methoxy group include a compound represented by the following formula (1).

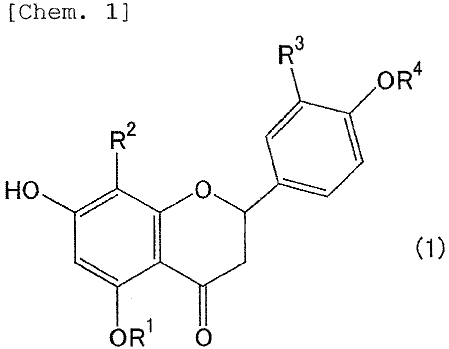

In the formula (1), R¹ represents a hydrogen atom or a methyl group. R² represents a hydrogen atom, a dimethylallyl group, or a geranyl group. R³ represents a hydrogen atom or a hydroxy group. R⁴ represents a hydrogen atom or a methyl group. At least one of R¹ or R⁴ represents a hydrogen atom.

Preferably, R² is a hydrogen atom or a dimethylallyl group.

Examples of the flavanone free of methoxy groups or having one methoxy group include isoxanthohumol, naringenin, hesperetin, and eriodictyol. Examples of the flavanone glycoside free of methoxy groups or having one methoxy group include isoxanthohumol glycosides, naringenin glycosides, hesperetin glycosides (e.g., hesperidin), and eriodictyol glycosides.

The flavanone compound in the present invention is preferably at least one compound selected from the group consisting of isoxanthohumol, an isoxanthohumol glycoside, naringenin, a naringenin glycoside, hesperetin, a hesperetin glycoside (e.g., hesperidin), eriodictyol, and an eriodictyol glycoside, more preferably at least one compound selected from the group consisting of isoxanthohumol, naringenin, hesperidin, and eriodictyol. These flavanones and flavanone glycosides have a high level of URAT1 inhibitory action. In particular, isoxanthohumol is still more preferred as the flavanone compound because it has a high level of URAT1 inhibitory action.

The flavanone compound in the present invention may be obtained from any origin and by any production method. The flavanone compound may be one derived from a natural product or a synthesized product. A commercial product may be used, if available.

Isoxanthohumol can be prepared, for example, through a process such as heating of a hop (*Humulus lupulus,* Cannabaceae) extract. Heating of a hop extract can produce isoxanthohumol in the extract. A hop extract is usually prepared through a process involving extraction of hop cones with a solvent and purification as needed. A hop extract can be obtained by a known preparation method. Hops can be extracted, for example, by a method that uses an ethanol solvent, which is used as a preparation method of a hop extract for beer brewing. A hop extract is commercially available, and a commercial hop extract can also be used. A hop extract is heated to produce isoxanthohumol preferably at 80°C to 140°C (more preferably 85°C to 100°C) for 15 minutes to 5 hours (more preferably 20 minutes to 3 hours). The hop extract is purified to prepare isoxanthohumol by a known method. Purification is performed by, for example, a method using HPLC or an absorption column or a precipitation method based on changes in solubility. Isoxanthohumol can also be produced by isomerizing xanthohumol by heating. Here, the heating temperature is preferably 80°C to 140°C (more preferably 85°C to 100°C) for 15 minutes to 5 hours (more preferably 20 minutes to 3 hours). Isoxanthohumol obtained by isomerization can be concentrated or purified by a known method (e.g., filtration, vacuum concentration, or lyophilization), as needed.

Isoxanthohumol has been reported to be stable even at a high temperature of 100°C, for example. The Food Sanitation Act defines sterilization conditions as the standard of soft drinks and the like. For example, soft drinks having a pH of 4.0 or higher (excluding those having a pH of 4.6 or higher and a water activity higher than 0.94) need to be heated at 85°C for 30 minutes. In view of conversion of ingredients in such a sterilization process, isoxanthohumol is considered to be highly suitable for beverage applications.

In one embodiment, the present invention can provide various functional foods, functional beverages, and the like that are thermally stable and that contribute to maintaining and improving the health.

Naringenin, hesperetin, eriodictyol, and glycosides thereof are contained in citrus fruits such as lemons, limes, grapefruits, Citrus *unshiu,* and *sudachi.* For example, an extract of any of these citrus fruits is obtained from their leaves, fruits, or peels used as materials with a polar solvent (e.g., methanol, ethanol, or water), and the extract is purified by chromatography or the like, whereby a naringenin glycoside, a hesperetin glycoside, or an eriodictyol glycoside can be obtained. Naringenin, hesperetin, or eriodictyol can be obtained as an enzyme-treated product obtained by treating an extract from any of the above materials with β-glucosidase, or as a product obtained by further purifying the enzyme-treated product. An aglycone such as naringenin, hesperetin, or eriodictyol is derived from a glucoside by the β-glucosidase treatment. Extraction from citrus fruit-derived materials, and purification and enzymatic treatment of such extracts can be performed by known methods.

In the present invention, the compositions of the present invention may contain, for example, an extract of a plant-derived material containing the flavanone compound as a source of the flavanone compound, as long as the effect of the present invention is achieved.

As shown in the examples, the flavanone and the flavanone glycoside (flavanone compound) which are free of methoxy groups or which have one methoxy group, such as isoxanthohumol, had an action to inhibit URAT1 activity (URAT1 inhibitory action). The flavanone compound in the present invention had a higher level of URAT1 inhibitory action than xanthohumol.

Inhibiting the activity of the urate transporter URAT1 promotes uric acid excretion from the urine. The flavanone compound in the present invention has the URAT1 inhibitory action, and is thus suitably used to inhibit URAT1 and to promote uric acid excretion. The flavanone compound in the present invention is used as an active ingredient of the composition for promoting uric acid excretion and the composition for inhibiting URAT1, and can be used to produce these compositions.

The composition for promoting uric acid excretion of the present invention can promote uric acid excretion by URAT1 inhibition. The composition for promoting uric acid excretion of the present invention is suitably used to promote uric acid excretion by URAT1 inhibition.

The present invention also encompasses a composition for lowering blood uric acid level which contains isoxanthohumol as an active ingredient.

Isoxanthohumol has excellent URAT1 inhibitory action. As described above, inhibiting the activity of the urate transporter URAT1 promotes uric acid excretion from the urine. This is expected to lower the blood uric acid level. The composition for lowering blood uric acid level of the present invention can be used to lower the blood uric acid level by promoting uric acid excretion.

The composition for promoting uric acid excretion, the composition for inhibiting URAT1, and the composition for lowering blood uric acid level of the present invention are also collectively referred to as "the composition for promoting uric acid excretion or the like".

The composition for promoting uric acid excretion or the like of the present invention is useful in prevention or amelioration of a condition or disease that can be prevented or ameliorated by promoting uric acid excretion or lowering the blood uric acid level. Examples of such a condition or disease include hyperuricemia, gout, and urinary stone. In one embodiment, the composition for lowering blood uric acid level of the present invention is used for preventing or ameliorating hyperuricemia and/or for preventing or ameliorating gout. Herein, the expression "prevention of a condition or disease" refers to preventing the onset of a condition or disease, delaying the onset of a condition or disease, reducing the incidence of a condition or disease, reducing the onset risk of a condition or disease, and the like. The expression "amelioration of a condition or disease" refers to helping a subject recover from a condition or disease, alleviating a symptom of a condition or disease, delaying or preventing the progress of a condition or disease, and the like.

The flavanone compound used in the present invention is a compound that is contained in natural products, foods, and/or beverages and that has been used in foods and beverages. Thus, daily intake of the flavanone compound, for example, is unlikely to cause any problems in terms of safety. The present invention can thus provide a composition for promoting uric acid excretion or the like which contains a highly safe component as an active ingredient.

The composition for promoting uric acid excretion or the like of the present invention is applicable for either therapeutic use (medical use) or non-therapeutic use (non-medical use).

The composition for promoting uric acid excretion or the like of the present invention can be provided, for example, as a food or beverage, a pharmaceutical or quasi-pharmaceutical product, or feed. The composition for promoting uric acid excretion or the like of the present invention may be a food or beverage, a pharmaceutical or quasi-pharmaceutical product, feed, or the like by itself for promoting uric acid excretion, inhibiting URAT1, or lowering blood uric acid level; or may be a material, a preparation, or the like to be added to such a product or the like.

For example, the composition for promoting uric acid excretion or the like may be provided as an agent or the like, but it is not limited thereto. The agent can be provided directly as a composition, or can be provided as a composition containing the agent. The composition for promoting uric acid excretion, the composition for inhibiting URAT1, and the composition for lowering blood uric acid level of the present invention can be regarded as a uric acid excretion promoter, a URAT1 inhibitor, and a blood uric acid level lowering agent, respectively. The composition for promoting uric acid excretion or the like of the present invention may be in the form of a solid, liquid, paste, or the like.

In one embodiment, preferably, the composition for promoting uric acid excretion or the like of the present invention is a composition for oral ingestion. The composition for oral ingestion may be a food or beverage, a pharmaceutical or quasi-pharmaceutical product for oral administration, or feed, preferably a food or beverage or a pharmaceutical product for oral administration, more preferably a food or beverage.

The composition for promoting uric acid excretion or the like of the present invention may contain optional additives and optional components, in addition to the flavanone compound, as long as the effect of the present invention is not impaired. Such additives and components can be selected according to the composition form or the like. Those that can be used generally in foods, beverages, pharmaceutical products, quasi-pharmaceutical products, feed, and the like can be used.

When the composition for promoting uric acid excretion or the like of the present invention is provided as a food or beverage, a pharmaceutical or quasi-pharmaceutical product, feed, or the like, any common method can be used for the production. In production of the composition for promoting uric acid excretion or the like of the present invention, the flavanone compound may be a purified compound. An extract of a plant-derived material containing the flavanone compound, for example, may be used as a source of the flavanone compound, as long as the effect of the present invention is achieved. The composition for promoting uric acid excretion or the like which contains isoxanthohumol can also be produced by, for example, using a hop extract and heating the hop extract.

For example, the composition for promoting uric acid excretion or the like of the present invention is provided as a food or beverage, a component usable in a food or beverage (e.g., a food material or an optional food additive) can be added to the flavanone compound to provide various types of foods or beverages. Non-limiting examples of the food or beverage include general foods and beverages, health foods, health beverages, foods with function claims, foods for specified health uses, and foods and beverages for the sick. Examples of the health foods, the foods with function claims, and the foods for specified health uses can be used in various forms of preparations such as fine granules, tablets, granules, powders, capsules, chewable tablets, syrups, liquids, and liquid foods.

In an exemplary preferred embodiment, the composition for promoting uric acid excretion or the like of the present invention is a beverage. For example, since isoxanthohumol is thermally stable, the composition for promoting uric acid excretion or the like which contains isoxanthohumol is suitable for a beverage.

The beverage may be a non-alcohol beverage or alcohol beverages. Examples of the non-alcohol beverage include tea-based beverages, coffee beverages, non-alcoholic beer-taste beverages, carbonated beverages, functional beverages, fruit and/or vegetable-based beverages, lactic beverages, soy milk beverages, and flavored water. In one embodiment, preferably, the composition for promoting uric acid excretion or the like of the present invention is such a non-alcohol beverage, or an alcohol beverage.

When the composition for promoting uric acid excretion or the like of the present invention is a tea-based beverage, preferably, it is a black tea beverage or sugarless tea beverage. Examples of the sugarless tea beverage include green tea beverages, oolong tea beverages, barley tea beverages, brown rice tea beverages, adlay tea beverages, and sugarless black tea beverages.

When the composition for promoting uric acid excretion or the like of the present invention is a coffee beverage, preferably, it is a packaged coffee beverage or liquid coffee.

The term "non-alcoholic beer-taste beverages" as used herein refers to carbonated beverages with beer-like flavors, which are usually non-fermented non-alcohol beverages, substantially free of alcohols. Here, the non-alcoholic beer-taste beverages do not exclude beverages containing a trace amount (undetectable amount) of alcohol.

When the composition for promoting uric acid excretion or the like of the present invention is a carbonated beverage, preferably, it is a cola-flavored beverage, a clear carbonated beverage, ginger ale, a fruit juice-based carbonated beverage, a milk-containing carbonated beverage, or a sugarless carbonated beverage.

When the composition for promoting uric acid excretion or the like of the present invention is a functional beverage, preferably, it is a sports drink, an energy drink, a health-supporting beverage, or a jelly drink pouch.

When the composition for promoting uric acid excretion or the like of the present invention is a fruit and/or vegetable-based beverage, preferably, it is a 100% fruit juice, a fruit-containing beverage, a soft drink with a low fruit juice content, a pulp-containing fruit juice, or a pulp-containing beverage.

When the composition for promoting uric acid excretion or the like of the present invention is a lactic beverage, preferably, it is milk, a yogurt drink, a lactic acid bacteria beverage, or a milk-containing soft drink.

When the composition for promoting uric acid excretion or the like of the present invention is a soy milk beverage, preferably, it is soy milk or a soybean beverage.

Examples of the alcohol beverage include beer, beer-based beverages, and alcohol beverages other than the beer and beer-based beverages.

When the composition for promoting uric acid excretion or the like of the present invention is a beer-based beverage, preferably, it is low-malt beer or a beer-like beverage.

When the composition for promoting uric acid excretion or the like of the present invention is an alcohol beverage other than the beer and beer-based beverages, preferably, it is *shochu,* a *shochu* highball, liqueur, a cocktail, a spirit, or a whisky.

The form of the beverage is not particularly limited. Examples include packaged beverages. Packages for the packaged beverages are not particularly limited. Packages in any form and of any material may be used. For example, any of the following commonly used packages can be used: metal packages such as aluminum cans and steel cans; resin containers such as plastic bottles; paper containers such as drink cartons; glass containers such as glass bottles; and wooden containers such as barrels. The beverages are filled and sealed in any of these packages, whereby packaged beverages can be obtained.

When the composition for promoting uric acid excretion or the like of the present invention is provided as a pharmaceutical or quasi-pharmaceutical product, various dosage forms of pharmaceutical or quasi-pharmaceutical products can be provided by, for example, adding a pharmacologically acceptable carrier, an optional additive, or the like to the flavanone compound. Such a carrier, an additive, or the like may be of any pharmacologically acceptable type that can be used in pharmaceutical or quasi-pharmaceutical products. Examples thereof include excipients, binders, disintegrants, lubricants, antioxidants, and colorants. One or more of these can be used. Preferably, the form of administration ) (intake) of the pharmaceutical or quasi-pharmaceutical product is oral administration in order to sufficiently obtain the effect of the present invention. When the composition for promoting uric acid excretion or the like is provided as a pharmaceutical product, preferably, it is a pharmaceutical product for oral administration. Examples of dosage forms of preparations for oral administration include liquids, tablets, powders, fine granules, granules, sugar-coated tablets, capsules, suspensions, emulsions, and chewable tablets. The pharmaceutical products may be for non-human animals.

When the composition for promoting uric acid excretion or the like of the present invention is provided as feed, for example, the feed can be provided by adding a component usable in feed to the flavanone compound. The feed includes feed additives. Examples of the feed include livestock feed; feed for small animals such as rats and mice; and pet foods for dogs, cats, and the like.

The flavanone compound content in the composition for promoting uric acid excretion or the like of the present invention can be suitably set according to the form of the composition or the like. In one embodiment, the flavanone compound content in the composition for promoting uric acid excretion or the like of the present invention, in terms of the total flavanone compound content, is preferably 0.0001 wt% or more, more preferably 0.001 wt% or more, and is preferably 90 wt% or less. In one embodiment, the total flavanone compound content in the composition is preferably 0.0001 to 90 wt%, more preferably 0.001 to 90 wt%. The total flavanone compound content is the total amount of flavanone compounds when the composition contains two or more flavanone compounds. In one embodiment, when the composition for promoting uric acid excretion or the like of the present invention is provided as a food or beverage, a pharmaceutical or quasi-pharmaceutical product, feed, or the like, the total flavanone compound content in the composition is preferably in the above ranges.

The flavanone compound content can be measured by a known method using, for example, high performance liquid chromatography (HPLC) or LC-MS/MS.

The composition for promoting uric acid excretion or the like of the present invention can be fed or administered by an appropriate method according to the form of the composition. In order to sufficiently obtain the effect of the present invention, preferably, the composition for promoting uric acid excretion or the like of the present invention is orally fed (orally administered).

The intake (dosage) of the composition for promoting uric acid excretion or the like of the present invention is not limited, as long as it is the amount (effective amount) that provides the uric acid excretion promoting effect, URAT1 inhibiting effect, or blood uric acid level lowering effect. The intake may be suitably set according to the dosage form, administration method, body weight of a subject, and the like. In one embodiment, when the composition for promoting uric acid excretion or the like of the present invention is orally fed or administered to a human (adult), the intake of the composition in terms of total intake of flavanone compound is preferably 1 to 200 mg, more preferably 5 to 60 mg, per 60 kg per day. The total intake of flavanone compound is the total intake of flavanone compounds when two or more flavanone compounds are fed. Intake of the above amount in one or more portions per day, for example, one to several times (e.g., two to three times) per day, is preferred. In one embodiment, the composition for promoting uric acid excretion or the like of the present invention may be a composition for oral administration to be used to feed or administer the above amount of the flavanone compound per 60 kg body weight per day to an adult.

A subject to be fed (administered) with the composition for promoting uric acid excretion or the like of the present invention is not limited. The subject is preferably a mammal (a human or non-human mammal), more preferably a human. The subject to be fed with the composition for promoting uric acid excretion or the like of the present invention is preferably, for example, one who needs or wants to promote uric acid excretion. The subject to be fed with the composition for lowering blood uric acid level of the present invention is preferably one who needs or wants to lower the blood uric acid level. Such subjects include not only hyperuricemic subjects with a high blood uric acid level but also those who want to prevent an increase in blood uric acid level, such as those whose blood uric acid levels are not high but who are concerned about an increase in blood uric acid level, those who want to prevent hyperuricemia, those who want to prevent gout, and those who have a high intake of foods and/or beverages which increase the blood uric acid level.

In one embodiment, the composition for promoting uric acid excretion or the like of the present invention can be used for a subject in healthy conditions in order to prevent a condition or disease that is expected to be ameliorated by promoting uric acid excretion.

The composition for promoting uric acid excretion or the like of the present invention may be labeled with one or more of the followings on its package, container, instructions, or the like: usage, types of active ingredients, the effects described above, directions (e.g., administration route and administration method), and the like. The composition for promoting uric acid excretion or the like of the present invention may be labeled as having the URAT1 inhibitory action or promoting uric acid excretion, or as having an action based on these actions. For example, the composition may be labeled as lowering the blood uric acid level.

The present invention also encompasses a method of promoting uric acid excretion, a method of inhibiting URAT1, and a method of lowering blood uric acid level, which are described below.

A method of promoting uric acid excretion, including:
administering or feeding at least one flavanone compound selected from the group consisting of a flavanone free of methoxy groups or having one methoxy group and a flavanone glycoside free of methoxy groups or having one methoxy group to a subject

A method of inhibiting URAT1, including:
administering or feeding at least one flavanone compound selected from the group consisting of a flavanone free of methoxy groups or having one methoxy group and a flavanone glycoside free of methoxy groups or having one methoxy group

A method of lowering blood uric acid level, including: administering or feeding isoxanthohumol to a subject

Each of these methods may be a therapeutic method or a non-therapeutic method. The "non-therapeutic" is a concept that does not include medical activities, i.e., a concept that does not include surgery, therapy, or diagnosis of humans. In these methods, a subject is preferably a mammal (a human or non-human mammal), more preferably a human.

The present invention also encompasses the following use and the like:
use of at least one flavanone compound for promoting uric acid excretion, the at least one flavanone compound being selected from the group consisting of a flavanone free of methoxy groups or having one methoxy group and a flavanone glycoside free of methoxy groups or having one methoxy group;
use of at least one flavanone compound for inhibiting URAT1, the at least one flavanone compound being selected from the group consisting of a flavanone free of methoxy groups or having one methoxy group and a flavanone glycoside free of methoxy groups or having one methoxy group; and
use of isoxanthohumol for lowering blood uric acid level.
Each of these uses may be therapeutic or non-therapeutic use. The use is preferably for a mammal (humans or non-human mammals), more preferably for humans.

In these methods and uses, it is sufficient as long as the flavanone compound in an amount that provides the uric acid excretion promoting effect, URAT1 inhibiting effect, or blood uric acid level lowering effect (i.e., effective amount) is fed or administered to a subject. The flavanone compound and its preferred embodiments are as described above for the composition for promoting uric acid excretion or the like. A preferred flavanone compound intake, a preferred subject to be administered with the flavanone compound, and the like are as described for the composition for promoting uric acid excretion or the like of the present invention. The flavanone compound may be directly administered or fed, or may be administered or fed in the form of a composition containing the flavanone compound. For example, the composition for promoting uric acid excretion or the like of the present invention described above may be fed or administered.

In one embodiment, the present invention also encompasses use of at least one flavanone compound for producing the composition for promoting uric acid excretion or the composition for inhibiting URAT1, the at least one flavanone compound being selected from the group consisting of a flavanone free of methoxy groups or having one methoxy group and a flavanone glycoside free of methoxy groups or having one methoxy group. The present invention also encompasses use of isoxanthohumol for producing the composition for lowering blood uric acid level. The flavanone compound, its preferred embodiments, and the like are as described above for the composition for promoting uric acid excretion or the like.

### EXAMPLES

The following provides examples that more specifically describe the present invention. The present invention is not limited to these examples.

### <Example 1>

Test substances were isoxanthohumol (Gifu Shellac Manufacturing Co., Ltd.), naringenin (Funakoshi Co., Ltd.), hesperidin (Tokyo Chemical Industry Co., Ltd.), and eriodictyol (Funakoshi Co., Ltd.).

Canine kidney-derived MDCK2 cells in which human URAT1 was stably expressed (Shin et al., Nephrology 16 (2011), 156-163) was washed with Hanks' Balanced Salt Solution (HBSS) (Nacalai Tesque, Inc.), and then cultured in HBSS containing the test substance and uric acid labeled with 14C for 10 minutes. Each test substance was dissolved in dimethyl sulfoxide (DMSO) and added to HBSS to give a test substance concentration of 1, 5, 10, 15, 20, 40, 60, 80, 100, or 200 µg/mL. Here, the DMSO concentration in HBSS was 1.5% or lower. Uric acid was added to HBSS to give a concentration of 20 µM. Subsequently, the cells were washed again with HBSS, and then dissolved in a 0.1 N sodium hydroxide aqueous solution. The radiation dose of 14C in the solution was measured by a liquid scintillation counter, and the uptake amount of uric acid into the cells was measured. A positive control was provided by adding benzbromarone at 30 µM. A test substance non-addition group as a control was provided by adding only DMSO to HBSS (DMSO concentration: 1%). The tests were performed with n = 3 for the control and for the test substances at each concentration. The inhibition rate (%) of URAT1 was calculated from the uptake amount of uric acid in each test substance addition group, taking the uptake amount of uric acid in the test substance non-addition group as 100%. The uptake amount of uric acid was determined with an average of n = 3. The calculation formula of the URAT1 inhibition rate (%) is as follows. The control group is the test substance non-addition group. Inhibition rate (%) = 100 × {((uptake amount of uric acid in control group) - (uptake amount of uric acid in test substance addition group))/uptake amount of uric acid in control group}

### <Comparative Example 1>

The inhibition rate (%) of URAT1 was determined by the same method as in Example 1, except that the test substance was xanthohumol (Alexis Biochem).

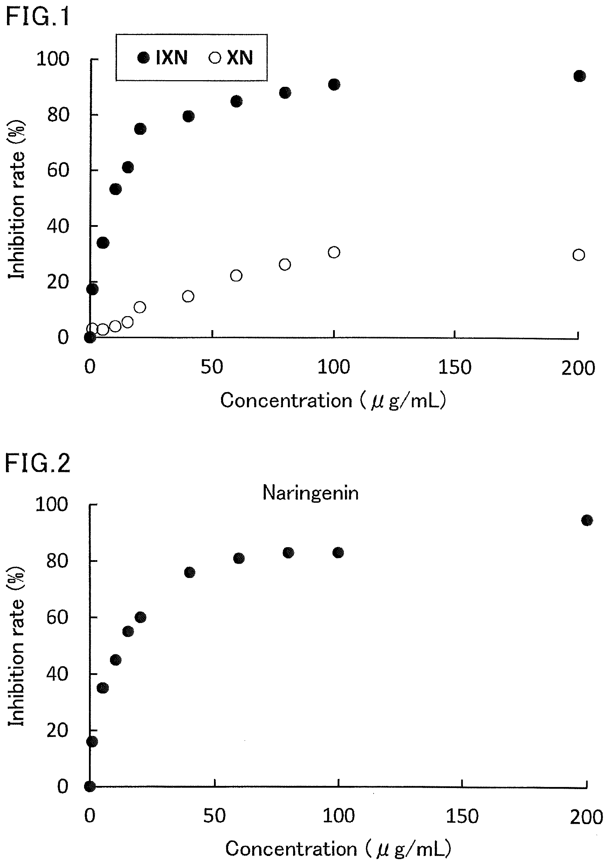

Figs. 1 to 4 show the evaluation results of the effects on URAT1 activity by the cultures of Example 1 and Comparative Example 1 to which the test substances were added individually. Fig. 1 is a graph showing the URAT1 inhibition rate of isoxanthohumol. Fig. 1 also shows the URAT1 inhibition rate of xanthohumol of Comparative Example 1 for comparison. In Fig. 1, IXN (black dots) represents isoxanthohumol; and XN (white dots) represents xanthohumol.

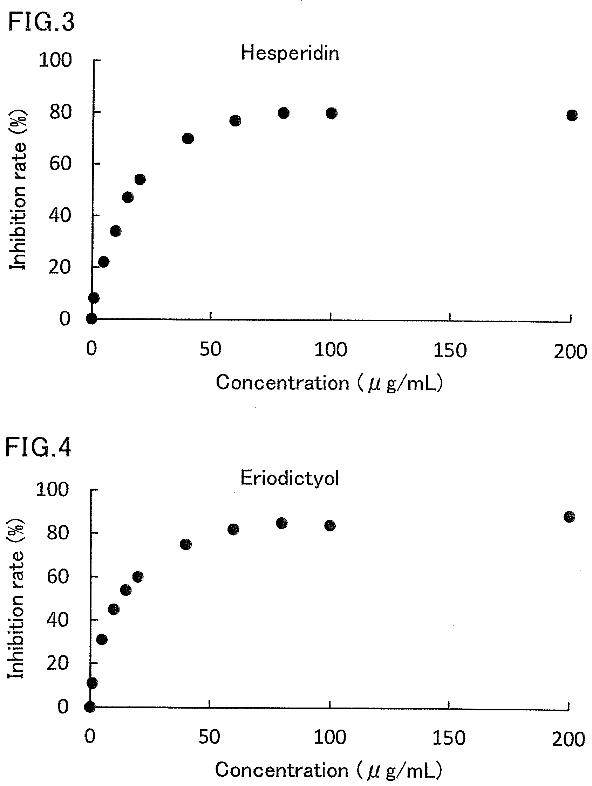

Fig. 2 is a graph showing the URAT1 inhibition rate of naringenin. Fig. 3 is a graph showing the URAT1 inhibition rate of hesperidin. Fig. 4 is a graph showing the URAT1 inhibition rate of eriodictyol.

Each culture to which the flavanone compound, i.e., isoxanthohumol, naringenin, hesperidin, or eriodictyol, was added showed a significant increase in the inhibition rate. In contrast, the culture to which xanthohumol, which is one of chalcones belonging to flavonoids, was added (Comparative Example 1) did not show a significant increase in the inhibition rate. The inhibition rate of benzbromarone (30 µM) was 99 to 100%. For each of the test substances used in Example 1, the test substance concentration (IC₅₀) at which URAT1 was inhibited 50% was calculated as an index of validity of biological or biochemical inhibitory action of each compound. Table 1 shows the calculation results of IC₅₀.

**[Table 1]**

| Test substance | IC₅₀ (µg/mL) |
|---|---|
| Isoxanthohumol | 8.90 |
| Naringenin | 11.40 |
| Hesperidin | 16.97 |
| Eriodictyol | 12.53 |

Of the test substances, isoxanthohumol showed the lowest IC₅₀, and was thus considered to have the highest level of URAT1 inhibition activity.

### INDUSTRIAL APPLICABILITY

The present invention is useful in the food and beverage field and the pharmaceutical field, for example.

## Claims

1. A composition for promoting uric acid excretion, comprising:
at least one flavanone compound selected from the group consisting of a flavanone free of methoxy groups or having one methoxy group and a flavanone glycoside free of methoxy groups or having one methoxy group, as an effective ingredient.

2. The composition for promoting uric acid excretion according to claim 1,
wherein the flavanone compound is at least one compound selected from the group consisting of isoxanthohumol, naringenin, hesperidin, and eriodictyol.

3. The composition for promoting uric acid excretion according to claim 1 or 2,
wherein the flavanone compound is isoxanthohumol.

4. The composition for promoting uric acid excretion according to any one of claims 1 to 3,
wherein the composition promotes uric acid excretion by URAT1 inhibition.

5. A composition for inhibiting URAT1, comprising:
at least one flavanone compound selected from the group consisting of a flavanone free of methoxy groups or having one methoxy group and a flavanone glycoside free of methoxy groups or having one methoxy group.

6. The composition for inhibiting URAT1 according to claim 5,
wherein the flavanone compound is at least one compound selected from the group consisting of isoxanthohumol, naringenin, hesperidin, and eriodictyol.

7. A composition for lowering blood uric acid level, comprising:
isoxanthohumol as an active ingredient.

8. The composition for lowering blood uric acid level according to claim 7,
wherein the composition is used for preventing or ameliorating hyperuricemia.

9. The composition for lowering blood uric acid level according to claim 7,
wherein the composition is used for preventing or ameliorating gout.

10. The composition according to any one of claims 1 to 9,
wherein the composition is a food or beverage or a pharmaceutical product.

11. A method of promoting uric acid excretion, comprising:
administering or feeding at least one flavanone compound selected from the group consisting of a flavanone free of methoxy groups or having one methoxy group and a flavanone glycoside free of methoxy groups or having one methoxy group to a subject.

12. A method of inhibiting URAT1, comprising:
administering or feeding at least one flavanone compound selected from the group consisting of a flavanone free of methoxy groups or having one methoxy group and a flavanone glycoside free of methoxy groups or having one methoxy group to a subject.

13. A method of lowering blood uric acid level, comprising:
administering or feeding isoxanthohumol to a subject.

14. Use of at least one flavanone compound for promoting uric acid excretion,
the at least one flavanone compound being selected from the group consisting of a flavanone free of methoxy groups or having one methoxy group and a flavanone glycoside free of methoxy groups or having one methoxy group.

15. Use of at least one flavanone compound for inhibiting URAT1,
the at least one flavanone compound being selected from the group consisting of a flavanone free of methoxy groups or having one methoxy group and a flavanone glycoside free of methoxy groups or having one methoxy group.

16. Use of isoxanthohumol for lowering blood uric acid level.
